# EUROPEAN PATENT SPECIFICATION

(11) **EP 1 983 896 B1**
(45) Date of publication and mention of the grant of the patent: **21.06.2017**
(21) Application number: 07712663.9
(22) Date of filing: 02.02.2007
(51) Int. Cl.: A61B 5/11, G06K 9/00, A61B 5/103, G06K 9/62, A61B 5/00

(54) **GAIT ANALYSIS**
GANGANALYSE
ANALYSE DE DEMARCHE

(30) Priority: 02.02.2006 GB 0602127
(43) Date of publication of application: 29.10.2008
(73) Proprietor: Imperial Innovations Limited, London SW7 2AZ (GB)
(72) Inventor: YANG, Guang-Zhong, Surrey KT19 7ND (GB); LO, Benny, London NW8 0HQ (GB)
(74) Representative: Hibbert, Juliet Jane Grace
(86) International application number: PCT/GB2007/000358
(87) International publication number: WO 2007/088374

(56) References cited:
- EP-A1- 0 549 081
- EP-A2- 0 816 986
- WO-A-01/88477
- WO-A-2004/073494
- JP-A- 2005 118 402
- US-A1- 2003 002 705
- US-A1- 2005 177 929
- US-A1- 2006 000 420
- KAVANAGH J J ET AL: "Coordination of head and trunk accelerations during walking" EUROPEAN JOURNAL OF APPLIED PHYSIOLOGY ;, SPRINGER-VERLAG, BE, vol. 94, no. 4, 1 July 2005 (2005-07-01), pages 468-475, XP019343496 ISSN: 1439-6327
- KAVANAGH J J ET AL: "Upper body accelerations during walking in healthy young and elderly men" GAIT & POSTURE, ELSEVIER, vol. 20, no. 3, December 2004 (2004-12), pages 291-298, XP004744630 ISSN: 0966-6362
- WAARSING J H ET AL: "Quantifying the stability of walking using accelerometers" ENGINEERING IN MEDICINE AND BIOLOGY SOCIETY, 1996. BRIDGING DISCIPLINES FOR BIOMEDICINE., 18TH ANNUAL INTERNATIONAL CONFERENCE OF THE IEEE AMSTERDAM, NETHERLANDS 31 OCT.-3 NOV. 1996, NEW YORK, NY, USA,IEEE, US, vol. 2, 31 October 1996 (1996-10-31), pages 469-470, XP010262084 ISBN: 0-7803-3811-1
- SABELMAN E ET AL: "Accelerometric activity identification for remote assessment of quality of movement" SAN FRANCISCO, CA, USA 1-5 SEPT. 2004, PISCATAWAY, NJ, USA,IEEE, US, 1 September 2004 (2004-09-01), pages 4781-4784Vol7, XP010775467 ISBN: 0-7803-8439-3

## Description

The present invention relates to a method and system of analysing gait.

In analysing gait it is often desirable to monitor gait patterns pervasively, that is in a subject's natural environments in contrast to relying on a subject walking on a treadmill in front of a video camera. Known pervasive gait analysis systems typically place sensors on the ankle, knee or waist of the subjects, aiming to capture the gait pattern from leg movements. However, due to variation in sensor placement, these systems often fail to provide accurate measurements or require extensive calibration for detecting predictable gait patterns, for example abnormal gait patterns following an injury.

The reader is directed to the articles by Kavanagh J J et al, "Coordination of head and trunk accelerations during walking", from European Journal Of Applied Physiology, Springer-Verlag, BE, vol. 94, no. 4, ISSN 1439-6327, pages 468 - 475 and "Upper body accelerations during walking in healthy young and elderly men", from GAIT & POSTURE, ELSEVIER, (200412), vol. 20, no. 3, ISSN 0966-6362, PAGE 291 - 298 and the approaches discussed in International patent application no. WO2004/073494, European patent application no. EP0816986, US patent application no. US2006/000420, US patent application no. US2003/002705 and European patent application no. EP0549081.

The inventors have made the surprising discovery that efficient gait analysis can be performed using an accelerometer placed on a subject's ear for example using an ear piece. Such an ear piece can be worn pervasively and can provide accurate measurements of the gait of the subject for gait analysis, for example in the study of recovery after injury or in sports investigations.

The invention is set out in the independent claims. Further, optional features of embodiments of the invention are set out in the remaining claims.

The analysis may include detecting certain types of gait patterns by comparing a signature derived from the sensed head acceleration to one or more base line signatures. It may also include monitoring the historical development of a gait pattern of a subject by storing signatures derived from the acceleration signals and compare future signatures against one or more of the stored signatures (the stored signatures thus acting as the baseline). The acceleration sensor senses head acceleration in a substantially vertical direction when the subject is in an upright position. This is believed to measure the shockwaves travelling through the spine to the head as the subject's feet impact on the ground during walking or running.

The acceleration sensor may be mounted on the head in a number of ways, for example in an ear piece to be placed inside the outer ear, a hearing-aid-type clip to be worn around and behind the ear, or an ear clip or ear ring to be worn on the ear lobe. Alternatively, in an unclaimed aspect of the current disclosure, the acceleration sensor may be secured to another form of head gear for example, a headband or a hat, a hearing aid or spectacles, and may in some applications be surgically implanted.

The signature can be derived from the acceleration signal using a number of techniques, for example a Fourier transform or wavelet analysis. The signature may be analysed in a number of ways including calculating its entropy, using it as an input to a self-organised map (SOM) or a spatio-temporal self-organised map (STSOM), as described in more detail below.

An exemplary embodiment of the invention is now described with reference to the attached drawings, in which:
Figures 1A to C schematically show a number of different ways of attaching the acceleration sensor to a subject's head;
Figures 2A to C show acceleration data obtained using an embodiment of the invention for a subject before and after injury and when recovered; and
Figures 3A to C show plots of the corresponding Fourier transform.

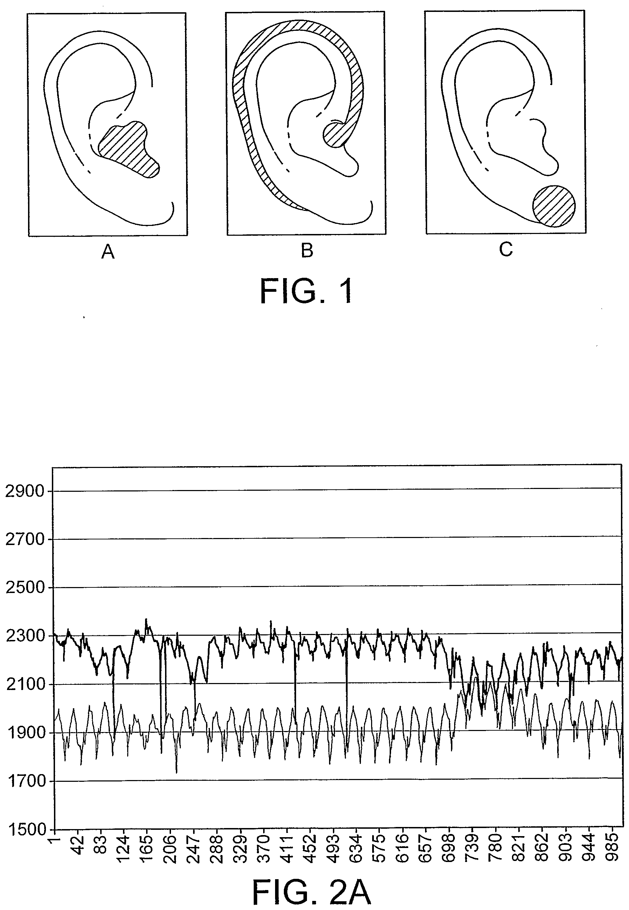

Figures 1A to C illustrate three different housings for an acceleration sensor to measure head acceleration (A: earplug; B: behind-the-ear clip; C: ear clip or ring). Inside the housing an acceleration sensor is provided, coupled to a means for transmitting the acceleration signal to a processing unit where it is analysed. Additionally, the housing may also house means for processing the acceleration signal, as described in more detail below. The result of this processing is then either transmitted to a processing unit for further processing or may be stored on a digital storage means such as a flash memory inside the housing. While Figures 1A-C show different ways of mounting an acceleration sensor to a subjects' ear, alternative means of mounting the sensor to the head are also envisaged, for example mounting on a headband or hat or integrated within a pair of spectacles or head phones.

The acceleration sensor may measure acceleration along one or more axes, for example one axis aligned with the horizontal and one axis aligned with the vertical when the subject is standing upright. Of course, a three axis accelerometer could be used, as well.

It is understood that the housing may also house further motion sensors such as a gyroscope or a ball or lever switch sensor. Furthermore, gait analysis using any type of motion sensor for detecting head motion is also envisaged.

Figures 2A to C show the output for each of two axes for such an acceleration sensor worn as described, with the dark trace showing the horizontal component and the lighter trace showing the vertical component. The y-axis of the graphs in Figures 2A to C shows the measured acceleration in arbitrary units and the x-axis denotes consecutive samples at a sampling rate of 50 Hz. As is clear from the cyclical nature of the traces, each of the figures shows several footstep cycles.

The present embodiment uses the vertical component of head acceleration (lighter traces in Figures 2A to C) to analyse gait. It is believed that this acceleration signal is representative of the shock wave travelling up the spine as the foot impacts the ground during walking or running. This shockwave has been found to be rich in information on the gait pattern of a subject.

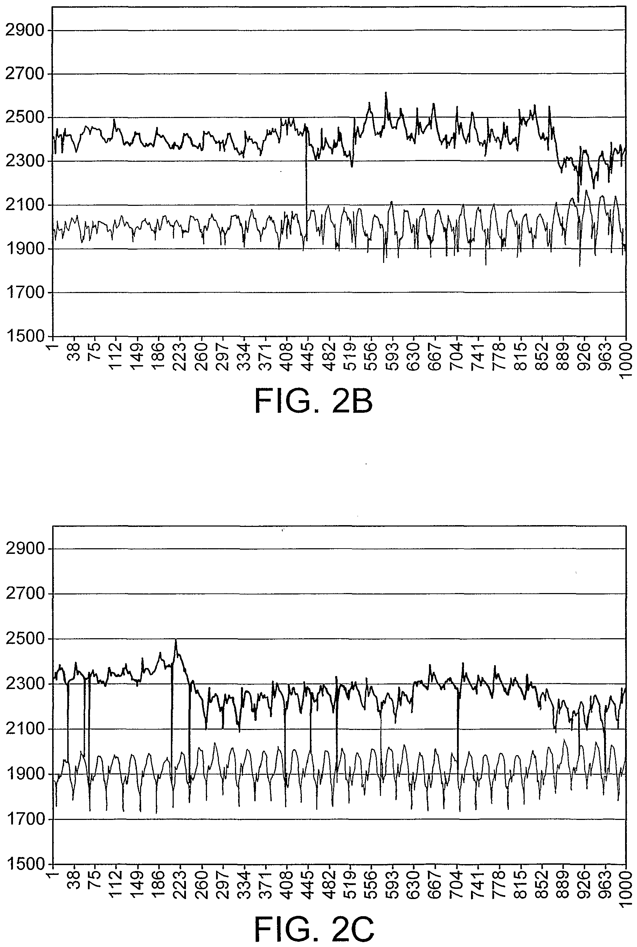

For example, in a healthy subject, gait patterns tend to be highly repetitive as can be seen in Figure 2A showing the acceleration traces for a healthy subject. By contrast, in Figure 2B, which shows acceleration traces of a subject following an ankle injury, it can be seen that following the injury the acceleration traces become much more variable, in particular for the vertical acceleration (lighter trace). It is believed that this is associated with protective behaviour while the subject walks on the injured leg, for example placing the foot down toes first rather than heel first followed by rolling of the foot as in normal walking.

Figure 2C shows acceleration traces from the same subject following recovery and it is clear that the repetitive nature of, in particular, the vertical acceleration trace that regularity has been restored.

Based on the above finding, the detection of a gait pattern representative of an injury (or, generally, the detection of a gait pattern different from a baseline gait pattern) may be achieved by suitable analysis of the above described acceleration signals. In one embodiment, the vertical acceleration signal is analysed using a Fourier transform for example, calculated using the Fast Fourier Transform (FFT) algorithm with a sliding window of 1024 samples. The abnormal gait pattern can then be detected from the frequency content.

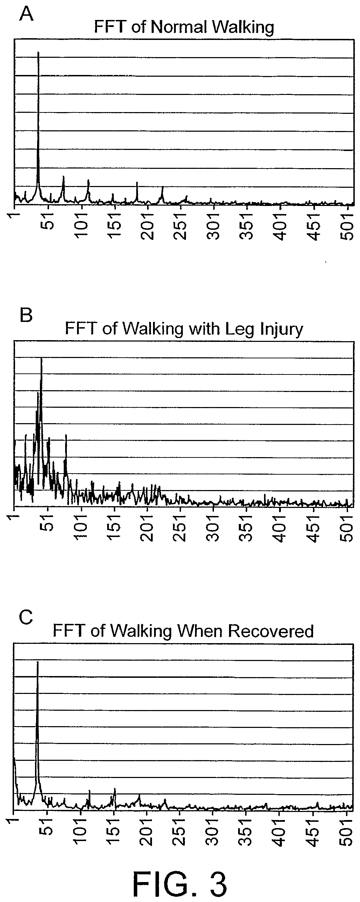

Figures 3A to C show the FFT for the respective acceleration measurements of Figures 2A to C. The y-axis is in arbitrary units and the x-axis is in units of (25/512) Hz, i.e. approximately 0.05 Hz. While the absolute value of the energy of the FFT (plotted along the y-axis) will depend on factors such as the exact orientation of the acceleration sensor with respect to the shockwave travelling through the spine and its placement on the head, as well as the overall pace of the gait, the plots clearly contain information on the type of gait pattern in the relative magnitudes of the energy of the FFT at different frequencies. It is clear that the relative magnitudes of the FFT peaks have changed.

As can be seen from Figure 3A, the FFT of the acceleration signal of a healthy subject shows a plurality of, decaying harmonics. By contrast, the leg injury data (Figure 3B) shows a much broader frequency content in which the spectrum lacks the well defined peaks of Figure 3A and the non-uniform harmonics indicate abnormal gait. Figure 3C shows the FFT of acceleration data for the same subject following recovery, and it can be seen that, to a large extent, the pre-injury pattern has been restored.

Summarising, a signature indicative of the gait pattern can be derived from the acceleration data and used to classify the gait pattern for example as normal or injured as above as demonstrated by the above data. In the above example, the signature is a Fourier transform. It is understood that other ways of calculating a signature are equally envisaged. For example, a signature can be calculated using wavelet analysis, for example by passing the data through a wavelet transform (e.g. first order Debauchies) and then using the transformed data as an input to a classifier, e.g. a SOM. For example, only the first high frequency component of the wavelet transfer could be used as an input to the classifier.

Once a signature is derived as described above, it can be analysed automatically in order to detect changes in the gait pattern. On the one hand, it may be desirable to detect whether the gait pattern is close to a desired gait pattern. This can be useful for example in training athletes. To this end, a signature obtained from acceleration data of a subject, for example an athlete, is obtained and compared to a baseline signature obtained from baseline data representing desired behaviour. The resulting information may then be used to, help an athlete in his training, for example helping a long distance runner to adjust his leg movements.

On the other hand, it may be desirable to use the above analysis to detect changes over time within a subject. For example, this can be useful in pervasive health monitoring where the gait pattern of a patient can be monitored such that a doctor or healthcare professional can be notified when a change in the gait pattern indicative of an injury is detected.

For example, one measure that can be used to detect changes in the signature is to calculate the entropy of the signature. In the example of the FFT described with reference to Figures 3A to C, it is clear that the entropy value for the injury data would be much larger than the entropy value for the normal data.

One way to compare and classify signatures is to use them as an input for a self organized map (SOM). For example, the energies of the FFT at the first four harmonics can be used as an input vector to an SOM. A person skilled in the art will be aware of the use of SOM for the analysis and clarification of data and the implementation of an SOM to analyse the signature as described above is well within the reach of normal skill of the person skilled in the art. Briefly, the SOM is presented with input vectors derived from the signatures described above during a training period for a sufficiently long time to allow the SOM to settle. Subsequently, activations of the output units of the SOM can then be used to classify the data. For example, it has been found that in a trained SOM data from the subject of Figures 2 and 3 may activate a first subset of units before injury and a second subset of units after injury.

In the embodiment described above, a signature is calculated using a sliding window FFT. As such, the resulting signature will be time varying such that more than one unit of an SOM will be activated over time. If it is desired to analyse the time varying nature of the input vector derived from the signature, an alternative analysis technique described in co-pending patent application WO2006/09773 may be used. The application describes an arrangement, referred to as Spatio-Temporal SOM (STSOM) below, of SOMs in which, depending on the measure of the temporal variation of the output of a first layer SOM, a second layer SOM is fed with a transformed input vector which measures the temporary variation of the features in the original input vector. As in a conventional SOM, the output of the second, temporal layer SOM can then be used to classify the data based on its temporal structure.

Briefly, classifying a data record using an STSOM involves:
(a) defining a selection variable indicative of the temporal variation of sensor signals within a time window;
(b) defining a selection criterion for the selection variable;
(c) comparing a value of the selection variable to the selection criterion to select an input representation for a self organising map and deriving an input from the data samples within the time window in accordance with the selected input representation; and
(d) applying the input to a self organising map corresponding to the selected input representation and classifying the data record based on a winning output unit of the self organising map.

For example, the selection variable may be calculated based on the temporal variability of the output units of a SOM.

Training an STSOM may involve:
(a) computing a derived representation representative of a temporal variation of the features of a dynamic data record within a time window;
(b) using the derived representation as an input for a second self - organised map; and
(c) updating the parameters of the self-organised map according to a training algorithm.

The training may involve the preliminary step of partitioning the training data into static and dynamic records based on a measure of temporal variation. Further details of training an STSOM and using it for classification can be found in the above-mentioned published patent application.

It is understood that the sensor signals of the above described embodiment may also be used for human posture analysis and/or activity recognition. Furthermore, the system described above could be an integral part of a body sensor network of sensing devices where multiple sensing devices distributed across the body are linked by wireless communication links.

## Claims

1. A method of analysing a gait of a human subject comprising:
measuring, using an acceleration sensor secured to an ear of the human subject, a signal representative of acceleration in at least a substantially vertical direction, when the human subject is in an upright position, of the head of the human subject whose gait is to be analysed;
using only the measured signal representative of acceleration in a substantially vertical direction, when the human subject is in an upright position, said signal being representative of a shockwave travelling thorough the spine of the human subject associated with gait, applying a transform to the measured signal of the acceleration sensor to compute a gait signature representative of the gait of the human subject; and
comparing the gait signature to a baseline signature to detect differences therebetween; wherein:
the baseline signature is representative of a healthy human subject, thereby detecting injury or recovery of the subject; or
one or more signatures are stored over time and then used as the baseline signature in order to monitor changes in the gait signature over time, thereby detecting injury or recovery of the human subject; or
the baseline signature is representative of a desired behavior of an athlete, thereby aiding in athletic training of the subject.

2. A method as claimed in claim 1 in which the transform is a Fourier transform, and optionally wherein the signature includes the values of the energy of the first n harmonics of the measured signal.

3. A method as claimed in claim 1 in which the transform is a wavelet analysis.

4. A method as claimed in any one of the preceding claims in which the gait signature is used as an input to a self organised map or a spatio-temporal self-organised map.

5. A method as claimed in any one of the preceding claims including calculating the entropy of the gait signature, and using the calculated entropy to compare the gait signature to the baseline signature.

6. A gait analysis system for analysing a gait of a human subject, the system comprising:
an acceleration sensor mounted in a housing which is adapted to be secured to an ear of the human subject, the acceleration sensor being arranged to measure acceleration in at least a substantially vertical direction, when the human subject is in an upright position, of the head of the human subject whose gait is to be analysed;
an analyser operatively coupled to the sensor and operable to receive the measured signal and using only the measured signal representative of acceleration in a substantially vertical direction, when the human subject is in an upright position, said signal being representative of a shockwave travelling thorough the spine of the human subject associated with gait, to apply a transform thereto for computing a gait signature representative of a gait pattern of the human subject; and
a comparator operable to compare the gait signature to a baseline signature in order to detect the differences therebetween;
wherein the housing is configured such that the output is representative of head acceleration in a substantially vertical direction when the human subject is in an upright position; wherein:
the baseline signature is representative of a healthy subject, thereby detecting injury or recovery of the subject; or
one or more signatures are stored over time and then used as the baseline signature in order to monitor changes in the gait signature over time, thereby detecting injury or recovery of the subject; or
the baseline signature is representative of a desired behavior of an athlete, thereby aiding in athletic training of the subject.

7. A system as claimed in claim 6 which further includes a memory for storing the one or more signatures over time as the baseline signature.

8. A system as claimed in claim 6 or 7, the system being included within the housing.

9. A system as claimed in any of claims 6 to 8 in which the housing includes an ear plug, a behind-the-ear clip, an ear ring, an ear clip, or a hearing aid.

10. A system as claimed in any one of claims 6 to 9, in which the transform is a Fourier transform, and optionally wherein the signature includes the values of the energy of the first n harmonics of the measured signal.

11. A system as claimed in any of claims 6 to 9 in which the transform is a wavelet analysis.

12. A system as claimed in any of claims 6 to 11 further including a further analyser including a self organised map or a spatio-temporal self organised map which is operable to receive the signature as an input.

## Patentansprüche

1. Verfahren zum Analysieren eines Gangs eines menschlichen Subjekts, aufweisend:
Messen, mit einem an einem Ohr des menschlichen Subjekts angebrachten Beschleunigungssensor, eines Signals, das eine Beschleunigung des Kopfes des menschlichen Subjekts, dessen Gang analysiert werden soll, in zumindest einer im Wesentlichen vertikalen Richtung darstellt, wenn sich das menschliche Subjekt in einer aufrechten Position befindet;
unter Verwendung nur des gemessenen Signals, das eine Beschleunigung in einer im Wesentlichen vertikalen Richtung darstellt, wenn sich das menschliche Subjekt in einer aufrechten Position befindet, wobei das Signal eine durch die Wirbelsäule des menschlichen Subjekts verlaufende Stoßwelle im Zusammenhang mit dem Gang darstellt, Anwenden einer Transformation auf das gemessene Signal des Beschleunigungssensors, um eine Gangsignatur zu berechnen, die den Gang des menschlichen Subjekts darstellt; und
Vergleichen der Gangsignatur mit einer Grundliniensignatur, um Unterschiede dazwischen zu erkennen; wobei:
die Grundliniensignatur ein gesundes menschliches Subjekt darstellt, sodass Verletzung oder Genesung des Subjekts erkannt werden; oder
eine oder mehrere Signaturen mit der Zeit gespeichert und dann als die Grundliniensignatur verwendet werden, um Änderungen der Gangsignatur mit der Zeit zu überwachen, sodass Verletzung oder Genesung des menschlichen Subjekts erkannt werden; oder
die Grundliniensignatur ein erwünschtes Verhalten eines Athleten darstellt, sodass sie beim athletischen Training des Subjekts hilft.

2. Verfahren nach Anspruch 1, wobei die Transformation eine Fourier-Transformation ist, und wobei die Signatur optional die Werte der Energie der ersten n Harmonischen des gemessenen Signals enthält.

3. Verfahren nach Anspruch 1, wobei die Transformation eine Wavelet-Analyse ist.

4. Verfahren nach einem der vorhergehenden Ansprüche, wobei die Gangsignatur als eine Eingabe für eine selbstorganisierte Karte oder eine räumlich-zeitliche selbstorganisierte Karte verwendet wird.

5. Verfahren nach einem der vorhergehenden Ansprüche, einschließlich Berechnen der Entropie der Gangsignatur und Verwenden der berechneten Entropie zum Vergleichen der Gangsignatur mit der Grundliniensignatur.

6. Ganganalyse-System zum Analysieren eines Gangs eines menschlichen Subjekts, wobei das System aufweist:
einen Beschleunigungssensor, montiert in einem Gehäuse, das dafür ausgelegt ist, an einem Ohr des menschlichen Subjekts angebracht zu werden, wobei der Beschleunigungssensor zum Messen einer Beschleunigung des Kopfes des menschlichen Subjekts, dessen Gang analysiert werden soll, in zumindest einer im Wesentlichen vertikalen Richtung, wenn sich das menschliche Subjekt in einer aufrechten Position befindet, angeordnet ist;
einen Analysator, der betrieblich mit dem Sensor verbunden ist und dafür betriebsfähig ist, das gemessene Signal zu empfangen und unter Verwendung nur des gemessenen Signals, das eine Beschleunigung in einer im Wesentlichen vertikalen Richtung darstellt, wenn sich das menschliche Subjekt in einer aufrechten Position befindet, wobei das Signal eine durch die Wirbelsäule des menschlichen Subjekts verlaufende Stoßwelle im Zusammenhang mit dem Gang darstellt, eine Transformation darauf anzuwenden, um eine Gangsignatur zu berechnen, die ein Gangmuster des menschlichen Subjekts darstellt; und
einen Komparator, der für das Vergleichen der Gangsignatur mit einer Grundliniensignatur betriebsfähig ist, um die Unterschiede dazwischen zu erkennen; wobei das Gehäuse derart konfiguriert ist, dass die Ausgabe eine Beschleunigung des Kopfes in einer im Wesentlichen vertikalen Richtung darstellt, wenn sich das menschliche Subjekt in einer aufrechten Position befindet, wobei:
die Grundliniensignatur ein gesundes Subjekt darstellt, sodass Verletzung oder Genesung des Subjekts erkannt werden; oder
eine oder mehrere Signaturen mit der Zeit gespeichert und dann als die Grundliniensignatur verwendet werden, um Änderungen der Gangsignatur mit der Zeit zu überwachen, sodass Verletzung oder Genesung des menschlichen Subjekts erkannt werden; oder
die Grundliniensignatur ein erwünschtes Verhalten eines Athleten darstellt, sodass sie beim athletischen Training des Subjekts hilft.

7. System nach Anspruch 6, das ferner einen Speicher zum Speichern der einen oder mehreren Signaturen mit der Zeit als die Grundliniensignatur enthält.

8. System nach Anspruch 6 oder 7, wobei das System im Gehäuse enthalten ist.

9. System nach einem der Ansprüche 6 bis 8, wobei das Gehäuse einen Ohrstöpsel, einen Clip für hinter dem Ohr, einen Ohrring, einen Ohrclip oder eine Hörhilfe beinhaltet.

10. System nach einem der Ansprüche 6 bis 9, wobei die Transformation eine Fourier-Transformation ist, und wobei die Signatur optional die Werte der Energie der ersten n Harmonischen des gemessenen Signals enthält.

11. System nach einem der Ansprüche 6 bis 9, wobei die Transformation eine Wavelet-Analyse ist.

12. System nach einem der Ansprüche 6 bis 11, ferner enthaltend einen weiteren Analysator, der eine selbstorganisierte Karte oder eine räumlich-zeitliche selbstorganisierte Karte enthält, die zum Empfangen der Signatur als eine Eingabe betriebsfähig ist.

## Revendications

1. Procédé d'analyse de la démarche d'un sujet humain, comprenant :
la mesure, en utilisant un capteur d'accélération fixé à une oreille du sujet humain, d'un signal représentatif d'une accélération dans au moins une direction essentiellement verticale quand le sujet humain est en position verticale, de la tête du sujet humain dont on analyse la démarche ;
l'utilisation du seul signal mesuré représentatif de l'accélération dans une direction essentiellement verticale quand le sujet humain est en position verticale, ledit signal étant représentatif d'une onde de choc traversant la colonne vertébrale du sujet humain associée à la démarche, en appliquant une transformée du signal mesurée du capteur d'accélération pour calculer une signature de démarche représentative de la démarche du sujet humain ; et
la comparaison de la signature de démarche à une signature de base pour détecter des différences avec elle, où :
la signature de base est représentative d'un sujet humain sain, ce qui permet ainsi de détecter des blessures ou une récupération chez le sujet ; ou
une ou plusieurs signatures sont mémorisées dans le temps puis utilisées comme signature de base pour contrôler des changements dans le temps dans la signature de démarche, ce qui permet ainsi de détecter des blessures ou une récupération chez le sujet ; ou
la signature de base est représentative d'un comportement désiré d'un athlète, ce qui aide l'entraînement athlétique du sujet.

2. Procédé selon la revendication 1, dans lequel la transformée est une transformée de Fourier, et où facultativement la signature inclut les valeurs de l'énergie des n premières harmoniques du signal mesuré.

3. Procédé selon la revendication 1, dans lequel la transformée est une analyse par ondelettes.

4. Procédé selon l'une quelconque des revendications précédentes, dans lequel la signature de posture sert d'entrée à une carte auto-adaptative ou à une carte auto-adaptative spatiotemporelle.

5. Procédé selon l'une quelconque des revendications précédentes, incluant le calcul de l'entropie de la signature de démarche et l'utilisation de l'entropie calculée pour comparer la signature de démarche à la signature de base.

6. Système d'analyse de démarche destiné à analyser la démarche d'un sujet humain, le système comprenant :
un capteur d'accélération monté dans un logement adapté pour être fixé à l'oreille d'un sujet humain, le capteur d'accélération étant conçu pour mesurer une accélération dans au moins une direction essentiellement verticale quand le sujet humain est en position verticale, de la tête du sujet humain dont on analyse la démarche ;
un analyseur couplé fonctionnellement au capteur et actionnable pour recevoir le signal mesuré et n'utiliser que le signal mesuré représentatif de l'accélération dans une direction essentiellement verticale quand le sujet humain est en position verticale, ledit signal étant représentatif d'une onde de choc traversant la colonne vertébrale du sujet humain associée à la démarche, afin d'y appliquer une transformée pour calculer une signature de démarche représentative d'un motif de démarche du sujet humain ; et
un comparateur actionnable pour comparer la signature de démarche à une signature de base afin de détecter des différences entre les deux ;
le logement étant conçu de façon que la sortie soit représentative de l'accélération de la tête dans une direction essentiellement verticale quand le sujet humain est en position verticale, où :
la signature de base est représentative d'un sujet humain sain, ce qui permet ainsi de détecter des blessures ou une récupération chez le sujet ; ou
une ou plusieurs signatures sont mémorisées dans le temps puis utilisées comme signature de base pour contrôler des changements dans le temps dans la signature de démarche, ce qui permet ainsi de détecter des blessures ou une récupération chez le sujet ; ou
la signature de base est représentative d'un comportement désiré d'un athlète, ce qui aide l'entraînement athlétique du sujet.

7. Système selon la revendication 6, incluant en outre une mémoire pour enregistrer au moins une signature dans le temps comme signature de base.

8. Système selon la revendication 6 ou 7, le système étant inclus à l'intérieur du logement.

9. Système selon l'une des revendications 6 à 8, dans lequel le logement inclut une oreillette, une pince pour pavillon, une oreillette, une pince à oreille ou une prothèse auditive.

10. Système selon l'une des revendications 6 à 9, dans lequel la transformée est une transformée de Fourier, et où facultativement la signature inclut les valeurs de l'énergie des n premières harmoniques du signal mesuré.

11. Système selon l'une des revendications 6 à 9, dans lequel la transformée est une analyse par ondelettes.

12. Système selon l'une des revendications 6 à 11, incluant un autre analyseur incluant une carte auto-adaptative ou à une carte auto-adaptative spatiotemporelle, actionnable pour recevoir la signature en entrée.
